# EUROPEAN PATENT APPLICATION

(11) **EP 1 321 142 A1**
(43) Date of publication of application: **25.06.2003**
(21) Application number: 01403339.3
(22) Date of filing: 21.12.2001
(51) Int. Cl.: A61K 31/404, A61K 9/20

(54) **Solid pharmaceutical composition for oral administration of Tegaserod**

(71) Applicant: Novartis AG, 4056 Basel (CH)
(72) Inventor: Vitzling, Christian, c/o Novartis AG, 4000 Basel (CH); Aubert, Jérome, c/o Novartis AG, 4000 Basel (CH)

(57) **Abstract**

A solid pharmaceutical composition for oral adminstration comprising tegaserod in base or salt form in an amount of up to 10% by weight a bulking agent in an amount of 70 to 90% by weight a disintegrant in an amount of less than 14% by weight a glidant and a lubricant,

## Description

### Field of the invention

The present invention relates to pharmaceutical compositions, in particular to compositions for administering a 5-HT₄-receptor partial agonist as active agent. More particularly, the present invention relates to pharmaceutical compositions for administering tegaserod and to processes for manufacturing such compositions.

### Background of the invention

Tegaserod (3-(5-methoxy-1H-indol-3-yl-methylene)-N-pentylcarbazimidamide) or a pharmaceutically acceptable salt thereof is known from EP 505322 and under the trade marks ZELMAC and ZELNORM. Published PCT Application WO 00/10526 describes tegaserod compositions, e.g. solid oral pharmaceutical compositions and use in anal incontinence.

Despite the merits of the above-mentioned compositions, there remains a need for more economic and stable compositions which can be formulated effectively.

### Summary of the invention

In one aspect this invention provides a solid pharmaceutical composition for oral adminstration comprising
a 5-HT₄ partial agonist in base or salt form in an amount of up to 10% by weight,
a diluent in an amount of 70 to 90% by weight, and
the disintegrant in an amount of less than 15% by weight,
wherein the amounts by weight are based on the total weight of the composition.

The term "disintegrant" is understood to mean a substance or mixture of substances which facilitates disintegration of the composition after administration in order that the active ingredient be released from the composition as efficiently as possible to allow for its rapid dissolution (see e.g. "Remington's Pharmaceutical Science" 18th edition (1990). The Theory and Practice of Industrial Pharmacy" Lachman et al. Lea & Febiger (1970)).

The active agent used in compositions according to the present invention is a serotonergic active agent acting on the gastro-intestinal system as partial agonist of the 5-HT₄ receptor. It is poorly soluble and acid sensitive and preferably in salt form, e.g., hydrogen maleate or hydrochloride or in free form.

5-HT₄ receptor partial agonists are useful for the prevention and treatment of gastro-intestinal motility disorders, e.g., Irritable Bowel Syndrome (IBS), Gastro-Esophageal Reflux Disease (GERD), Functional Dyspepsia (FD), Post Operative Ileus (POI), Diabetic gastroporesis and chronic constipution.

A preferred agent is tegaserod, a 5-HT₄ partial agonist of formula 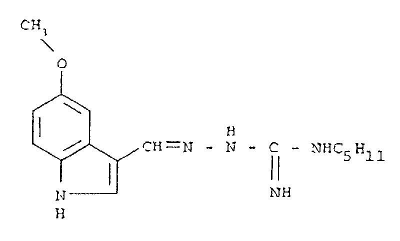 or pharmaceutically acceptable salt form thereof, e.g. the hydrogen maleate (hereinafter "hml") salt. Tegaserod has a solubility of about 0.02% at 25°C in water and is acid sensitive. We have found that compositions thereof may be produced which provide good absorption even in the stomach.

In one embodiment, the composition of the invention comprises less than 15%, e.g. less than 14% or 12% or less, e.g. 10% or less, e.g. 5 to 10% by weight of disintegrant based on the total weight of the composition. We have observed that the use of such a small percentage of disintegrant improves the dissolution rate.

The diluent may comprise lactose, mannitol, sucrose, calcium sulphate, calcium phosphate or microcrystalline cellulose (MCC USP (AvicelTM PH-102, FMC Corp.) The diluent may be present in an amount from 50 to 90 %, preferably from 70 to 90 % more preferably from 75 to 85%. Preferably the diluent is lactose consisting of α-lactose monohydrate and amorphous material (Spray dried lactoseTM, Formost Corp.).

As disintegrant the composition of the present invention may comprise:
- crospovidone (molecular weight >10⁶ Daltons), e.g. Polyplasdone XL®, Kollidon CL®, Polyplasdone XL-10®,
- pregelatinized starch (MW 30000-120000 Daltons), e.g., starch 1500™ (Colorcon UK).

Preferably, the disintegrant Is crospovidone which is preferably water insoluble. Ideally the disintegrant rapidly exhibits high capillary or pronounced hydration capacity with little tendency to gel formation.
The particle size of the disintegrant may be from about 1 to 500 micrometers. A preferred particle size distribution is from 10 to 400 e.g. less than 400 micrometers, e.g., for Polyplasdone XL® less than 80 micrometers, e.g., less than 74 micrometers for, e.g., Polyplasdone XL-10®, approximately 50% greater than 50 micrometers and maximum of 1% greater than 250 micrometers in size for, e.g., Kollidon CL®. A preferred crospovidone is Polyplasdone XL®, e.g., with a density of about 0.213 g/cm³ (bulk) or 0.273 g/cm³ (tapped).

The composition of the present invention may further comprise a glidant e.g. Colloidal silicon dioxide (Aerosil, Degussa).

The composition may further comprise one or more lubricants, e.g., in an amount within the range of from 3 to 7%, e.g. from 4 to 6% by weight of the composition.

Examples of such lubricants include:
- magnesium stearate (Faci),
- sodium benzoate
- glyceryl mono fatty acid, e.g. having a molecular weight of from 200 to 800 Daltons e.g. gylceryl monostearate (e.g., Danisco, UK),
- glyceryl dibehenate (e.g., CompritolATO888™, Gattefossé France)
- glyceryl palmito-stearic ester (e.g. Precirol™, Gattefossé France)
- polyoxyethylene glycol (PEG, BASF)
- hydrogenated cotton seed oil (Lubitrab, Edward Mendell Co Inc),
- castor seed oil (Cutina HR, Henkel)

In a preferred embodiment the lubricant is glyceryl dibehenate. We have observed that the use of glyceryl dibehenate improves lubrication properties and avoids tablet adhesion.

Further there is no or negligible impact on tegaserod in vitro dissolution rate and tablet disintegration of the composition.

The composition of the invention may comprise one or more binders, e.g., in an amount in the range of from 1 to 10%, e.g., 2 to 8%, e.g. 3% by weight. Particularly the following binders may be used:
- hydroxy-propyl-methyl cellulose (HPMC2910, Pharmacoat603TM, Shin-Etsu Chemical Co Ltd)
- copolyvidone (KollidonTM VA64, BASF)
- potato starch, wheat starch, com starch, e.g., having a molecular weight of from 30000 to 120000,
or a mixture thereof.

Other conventional exciplents which may optionally be present in the composition of the invention include preservatives, stabilisers, anti-adherents or silica flow conditioners or glidants, e.g., silicon dioxide (e.g., Syloid®, Aerosil®) as well as FD&C colours such as ferric oxides.

Other excipients disclosed in the literature, as for instance in Fiedler's "Lexicon der Hilfstoffe", 4th Edition, ECV Aulendorf 1996 and "Handbook of Pharmaceutical Excipients" Wade and Weller Ed.(1994), the contents of which are incorporated herein by reference, may be used in the pharmaceutical compositions according to the invention.

A preferred composition of the invention may comprise from about 0.5 to 15% by weight of tegaserod, less than 15 % by weight of disintegrant e.g. crospovidone, from 3 to 7% by weight of lubricant, e.g. glyceryl dibehenate, from 50 to 90% by weight of diluent, e.g. lactose, from 0.1 % to 1% by weight of glidant and optionally from 1 to 10% of binder, e.g. hydroxypropylmethyl cellulose (HPMC)

The compositions of this invention may be free or substantially free of surfactant.

In a further aspect the present Invention provides an oral, e.g. tablet composition comprising the active agent tegaserod.

Daily dosages required in practising the method of the present invention will vary depending upon, for example the mode of administration and the severity of the condition to be treated. An indicated daily dose is in the range of from about 1 to about 30 mg of active agent for oral use, conveniently administered once or in divided dosages.

In one embodiment the present invention provides a round shaped tablet with a diameter of 6 to 9 mm, preferably 7 mm.

In a further aspect the present invention provides a process for the production of the compositions of the invention. The compositions of the invention may be prepared by working up active agent with excipients. The following processes A,B and C are contemplated:

### Process A

The composition of the invention may be obtained by
(i) preparing a mixture of tegaserod, diluent and lubricant,
(ii) sieving the mixture
(iii) adding the disintegrant, glidant, lubricant and optionally binder and blending the sieved mixture of step (ii) and
(iv) forming tablets by direct compression.

Part of the lubricant may be added in mixture of step (i), the rest in the final mixture of step (iii) or the total amount of lubricant may be added in the final mixture of step (iii).

The resulting powder blends of step iii) are compressed on either a single punch press (Korsh EKO), 6 station-rotary press (Korsh PH106), 17 station-rotary press (Korsh PH 230) or 43 station-rotary press (Fette PT2090).

All components may be mixed together, sieved through and mixed again. Tablets are then formed by direct compression.

### Process B

The compositions of the invention may be obtained by
(i) preparing a mixture of Tegaserod and diluent,
(ii) sieving the mixture,
(iii) adding the disintegrant, glidant and optionally binder and blending the sieved mixture of step (ii), and
(iv) adding the lubricant by spray lubrication when forming tablets by direct compression.

A 43 station rotary press (Fette PT 2090) with a magnesium stearate spraying system may be conveniently used to carry out step (iv).

The components may be mixed together, sieved and mixed again. The lubricant is added by spray lubrication when the tablets are formed by direct compression.

### Process C

In another embodiment the compositions of the invention may be obtained by
(i) preparing a mixture of Tegaserod, diluent, disintegrant, glidant and optionally binder,
(ii) compacting the mixture of step (i) by roller compaction,
(iii) milling the mixture of step (ii), and
(iv) forming tablets by compression or adding the lubricant by spray lubrication when forming tablets by direct compression.

Tablets may be formed by compressing the resulting powder on a single punch press (Korsh EKO), 6 station-rotary press (Korsh PH106), 17 station-rotary press (Korsh PH 230), a 43 station-rotary press (Fette PT2090) or a 43 station rotary press (Fette PT 2090) with the magnesium stearate spraying system.

Following Is a description by way of example only of compositions and processes of the invention.

### Example 1

A 6 mg tablet is prepared using the direct compression method.

| Component | Quantitiy (125 mg tablet) % w/w |
|---|---|
| Tegaserod maleate | 6.65 |
| Lactose spray dried | 82.85 |
| Crospovidone | 6.00 |
| Aérosil | 0.50 |
| Compritol ATO 800 | 4.00 |

A blend is formed by mixing tegaserod maleate, lactose, crospovidone, aérosil and compritol. This blend is sieved and the mixture is blended again. The resulting powder blends are compressed using a 17 station-rotary press (Korsh PH 230) equipped with 7 mm, round upper punches.

### Example 2

A 6 mg tablet is prepared using the direct compression method with *in situ spray* lubrication.

| Component | Quantitiy (125 mg tablet) % w/w |
|---|---|
| Tegaserod maleate | 6.65 |
| Lactose spray dried | 84.85 |
| HPMC | 3.00 |
| Crospovidone | 5.00 |
| Aérosil | 0.50 |
| Magnesium stearate | < 0.3 |

A blend is formed by mixing tegaserod maleate, lactose, crospovidone, aérosil and compritol. This blend and the mixture is blended again. The lubricant magnesium stearate is added by spray lubrication. The resulting powder blends are compressed using a 43 station-rotary press (Fette PT 2090) equipped with 7 mm, round upper punches.

### Example 3

A 6 mg tablet is prepared using roller compaction

| Component | Quantitiy (125 mg tablet) % w/w |
|---|---|
| Tegaserod maleate | 6.65 |
| Lactose spray dried | 76.85 |
| Crospovidone | 10.00 |
| Aerosil | 0.50 |
| Compritol ATO 888 | < 0.3 |

Compositions are prepared by mixing tegaserod maleate, lactose, crospovidone, aerosil and compritol. This mixture is compacted by roller compaction and milled. Tablets are formed by compression.

The present invention this provides a solid oral pharmaceutical composition comprising a 5-HT₄-receptor partial agonist and a lower amount of disintegrant than witherto used.

This invention provides tegaserod compositions with fewer components than hitherto known and a simple dry process without granulation. The formulations of the present invention are less hygroscopic, overcome adhesion problems and provide complete or substantially complete dissolution within 30 minutes.

## Claims

1. A solid pharmaceutical composition for oral adminstration comprising
a 5-HT₄ partial agonist in base or salt form in an amount of up to 10% by weight,
a diluent in an amount of 70 to 90% by weight,
a disintegrant in an amount of less than 15% by weight, and
wherein the amounts by weight are based on the total weight of the composition.

2. A composition as claimed in claim 1 wherein the 5-HT₄ partial agonist is tegaserod.

3. A composition as claimed in claim 1 or 2 further comprising a glidant.

4. A composition as claimed in any preceding claim further comprising a lubricant.

5. A composition as claimed in any preceding claim further comprising a binder.

6. A composition as claimed in any of claims 2 - 5 wherein tegaserod is in the form of the maleate salt.

7. A composition as claimed in any preceding claim wherein the diluent is selected from the group consisting of lactose, mannitol, sucrose, calcium phosphate or microcrystalline cellulose.

8. A composition as claimed In any preceding claim wherein the diluent is lactose.

9. A composition as claimed in any preceding claim wherein the disintegrant is present in an amount less than 10% by weight based on the total weight of the compostion

10. A composition as claimed in any preceding claim wherein the disintegrant is crospovidone.

11. A pharmaceutical composition as claimed in any of claims 4 to 10 claim wherein the lubricant is present in an amount of 3 to 7% based on the total weight of the composition.

12. A pharmaceutical composition as claimed in any of claims 4 to 11 wherein the lubricant is glycerol monostearate or glycerol behenate.

13. A pharmaceutical composition as claimed in any of claims 3 to 12 wherein the glidant is colloidal silica dioxide.

14. A process for the production of a composition as claimed In any preceding claim which process is carried out under substantially dry conditions using granulation.

15. A process for the production of a composition as claimed in any preceding claim which process comprises:
(i) preparing a mixture of 5-HT₄-partial agonist, e.g. tegaserod, diluent and lubricant,
(ii) sieving the mixture,
(iii) adding the disintegrant, glidant and optionally binder and blending the sieved mixture of step (ii), and
(iv) forming tablets by direct compression.

16. A process for the production of a composition as claimed in claim 5 wherein the components are mixed with tegaserod, sieved and mixed again before tabletting.

17. A process for the production of a composition as claimed in any of claims 1 to 13 which process comprises:
(i) preparing a mixture of 5-HT₄ partial agonist, e.g. tegaserod, and diluent
(ii) sieving the mixture
(iii) adding the disintegrant, glidant and optionally binder and blending the sieved mixture of step (ii)
(iv) adding the lubricant by spray lubrication when forming tablets by direct compression.

18. A process for the production of a composition as claimed in claim 17 wherein all the components are mixed with tegaserod, sieved through and mixed again before tabletting.

19. A process for the production of a composition as claimed in any of claims 1 to 13 which process comprises:
(i) preparing a mixture of 5-HT₄ partial agonist e.g. tegaserod, diluent, disintegrant, glidant and optionally binder
(ii) compacting the premix of step (i) by roller compaction
(iii) milling the mixture of step (ii) and
(iv) forming tablets by compression.

20. A solid pharmaceutical composition for oral adminstration consisting of or consisting essentially of
tegaserod in base or salt form in an amount of up to10% by weight,
a diluent in an amount of 70 to 90% by weight,
a disintegrant in an amount of less than 15% by weight, e.g. 2 to 10%
a glidant and a lubricant,
wherein the amounts are by weight based on the total weight of the composition.
